Europäisches Patentamt

(19) European Patent Office

Office européen des brevets

(11) Publication number: **0 052 874**
A1

(12) **EUROPEAN PATENT APPLICATION**

(21) Application number: **81109808.6**

(22) Date of filing: **20.11.81**

(51) Int. Cl.³: **A 61 K 35/14**
**A 61 K 37/14**

(30) Priority: **21.11.80 US 208921**

(43) Date of publication of application:
**02.06.82 Bulletin 82/22**

(84) Designated Contracting States:
**BE CH DE FR GB IT LI NL SE**

(71) Applicant: **Marx, Gerard**
**7 Mercer Street**
**New York New York(US)**

(72) Inventor: **Marx, Gerard**
**7 Mercer Street**
**New York New York(US)**

(74) Representative: **Patentanwälte Grünecker,**
**Dr.Kinkeldey Dr.Stockmair, Dr.Schumann,Jakob,**
**Dr.Bezold Meister, Hilgers, Dr.Meyer-Plath**
**Maximilianstrasse 43**
**D-8000 München 22(DE)**

(54) **Method for synthesizing procoagulant factor VIII activity.**

(57) Disclosed herein is a method for synthesizing pro-coagulant factor VIII from inactive protein by incubating the protein in the presence of an effective amount for synthesizing procoagulant factor VIII of a manganese salt. The method is useful to synthesize AHF in plasma that has been collected with an anticoagulating agent.

EP 0 052 874 A1

METHOD FOR SYNTHESIZING
PROCOAGULANT FACTOR VIII ACTIVITY

The present invention pertains to a method of generating factor VIII, or anti-hemophilic factor (AHF) activity from inactive protein.

Anti-hemophilic factor (AHF), often referred to as factor VIII, is a protein material present in trace amounts in normal human and animal blood plasma. Factor VIII corrects the coagulation defect of hemophilic plasma and also a functional defect in VonWillebrand's disease plasma. Factor VIII is defined by its activity which is diminished in patients with hemophilia A. AHF is used for the treatment of hemophilia A (which is defined to be a deficiency of factor VIII procoagulant activity). One unit of factor VIII activity is defined as the amount in one mililiter of pooled, fresh, normal, citrate-treated plasma.

The basis for therapy of bleeding episodes in patients afflicted with hemophilia involves transfusion of material containing factor VIII procoagulant activity which temporarily corrects this specific defect. In general, whole blood is not administered to the patient, and its use is limited to restoration of blood volume in instances of severe loss. While plasma with a high factor VIII content may be employed, the preferred method of treatment involves administration of factor VIII concentrates.

Factor VIII is obtained from whole blood plasma donated by human volunteers. However, because only a small quantity of factor VIII is present in a given volume of blood, it is important to maximize the recovery of this protein material. The problem is complicated because whole blood is generally collected into anti-coagulant materials such as sodium citrate, sodium oxalate, citrate dextrose, citrate phosphate dextrose, or EDTA (ethylene – diamine tetraacetic acid). The presence of these anticoagulants tends to diminish the factor VIII activity present in the collected plasma. These standard anti-coagulant compounds are metal chelating agents and it has been postulated that they remove a metal element, responsible for factor VIII activity, from the plasma. The effects of these metal chelating anti-coagulant agents are cumulative, with the result that factor VIII activity constantly diminishes in their presence with the passage of time. Thus, if a unit of citrated blood is stored for more than a few days, under refrigeration, a substantial percentage of the factor VIII activity is lost. Although the coagulant activity of factor VIII is markedly increased or activated in the presence of traces of thrombin and other proteolytic enzymes such as trypsin, the increased activity is relatively short-lived. The thrombin activation effect cannot be observed in coagulant inactive material.

In general, whole blood collected from human volunteers in the presence of an anti-coagulant. By the time the blood is centrifuged, frozen, stored, and used for fractionation to manufacture a cryoprecipitate containing factor VIII, the labile factor VIII activity has diminished to approximately half, or less, of the original activity level present in freshly collected plasma. This makes it necessary to fractionate larger quantities of blood to obtain a single unit of factor VIII activity. The art has long sought a technique for enhancing the yield of factor VIII activity in blood plasma and particularly in the cryoprecipitate that normally serves as the vehicle for administration of factor VIII to humans.

It is an object of this invention to provide a method for generating factor VIII activity from inactive protein.

Another object of the present invention is to provide a method for generating factor VIII activity in plasma by incubation of such material with a divalent manganese salt.

A further aspect of the present invention is to provide a method for generating factor VIII activity in plasma cryoprecipitates by incubating the cryoprecipitate with a divalent manganese salt.

A still further object of the present invention is to provide a new product comprising a plasma cryoprecipitate containing an effective amount for generating factor VIII activity of a divalent manganese salt.

The foregoing objects, together with further aspects of the present invention, will be apparent upon consideration of the following specification.

It has long been reported that incubation of normal plasma with sodium citrate, ethylene diamine tetra-acetic acid (EDTA) and other metal chelating anticoagulants renders the plasma deficient in factor VIII (see Casillas et al. "Artificial Substrate for the Assay of Factors V and VIII", Coagulation, Volume 4, pages 107-111 (1971), and J.W. Bloom et al., "A Rapid Technique for the Preparation of Factor V Deficient Plasma", Thrombosis Res., Volume 15, page 595-599 (1979)).

Because of its property as a metal chelating agent, EDTA is commonly used as an anti-coagulant in the collection of fresh human and animal blood. The metal chelating action is considered to be necessary to remove calcium in whose absence the blood clotting reactions do not occur. Whole blood is collected into anti-coagulant citrate dextrose, anti-coagulant citrate phosphate dextrose, sodium citrate, and sodium oxalate. Although anti-coagulant heparin is not a metal chelating agent and acts through other mechanisms to prevent clotting, existing practice and regulation does not permit its use when only plasma (as contrasted with whole blood) is to be collected. A further drawback is that the lifespan of red blood cells in heparinized blood is somewhat diminished as contrasted with other anti-coagulant agents.

At the present time, sodium citrate is the anti-coagulant most commonly used for plasmapheresis. Exposure of whole blood to the presence of the above-mentioned metal chelating anti-coagulant agents successfully removes calcium (++) and prevents clotting. However, such exposure also results in depleting or destroying factor VIII activity. For this reason, it has long been thought that factor VIII procoagulant activity was dependent on the presence of calcium. However, efforts to regenerate such factor VIII activity in whole plasma, or in plasma derivatives (e.g. cryoprecipitate), by the addition of calcium salts have been wholly unsuccessful.

It has now been unexpectedly discovered that factor VIII is a metallo-protein containing manganese and that manganese salts can be used to generate factor VIII activity from plasma proteins which do not display such procoagulant activity. This is surprising because calcium has long been considered the cation responsible for factor VIII activity. In practicing the present invention it is preferred to incubate a plasma cryoprecipitate whose AHF activity level has been depleted by exposure to a metal chelating anti-coagulant, in the presence of a manganese salt. Suitable salts include the manganese halides, which are preferred, (i.e., chloride (especially preferred), bromide, iodide, fluoride), acetate, formate, citrate, oxalate, phosphate, nitrate, and sulphate. The list is not all inclusive and any pharmaceutically acceptable manganese salt may be employed in practicing the invention.

It has been determined that manganese salt incubation for factor VIII procoagulant activity synthesis is best conducted in the range of between about pH3 and pH8.5, and preferable between about pH6 and pH7.5. The temperature at which the incubation is conducted is not critical, and successful pro-coagulant activity synthesis has been obtained between about 4 and about 60 degrees centigrade, although optimum results have been obtained in the range of between about 25 and 45 degrees centigrade. As a general rule, incubation is preferably carried out at 37 degrees centigrade. Small quantities of inorganic salt buffering agents such as potassium chloride and sodium chloride are generally included in the incubation solution, but are not required. Preferably these agents are present in the range up to about 0.5 molar concentration. Inorganic bufferin agents including by way of non-limiting example phosphates, citrates, oxalates, barbiturates, imidazoles, borates, acetates, formates, succinates, and other pharmaceutically acceptable buffering agents of the type conventionally employed in plasma chemistry may also be present in the manganese incubated blood fraction in a similar concentration range.

Because AHF is only present in trace amounts in normal plasma, it would be necessary to administer large quantities of plasma to a patient in order to provide a therapeutic quantity of factor VIII. The likelihood of inducing an adverse immunologic reaction with antibodies carried in the patient's blood, taken together with the amplified risk of hepatitis attendant to such treatment, make it generally preferable to administer factor VIII in the form of a cryoprecipitate.

Cryoprecipitation is the principle technique employed for the large scale production of AHF concentrates. In this technique, fresh frozen plasma is pooled, thawed at 2 degrees centigrade and the cryoprecipitate collected. The cryoprecipitate (a viscous liquid material) is generally extracted with a buffer (of the type described above) sometimes followed by adsorption with aluminum hydroxide to remove prothrombin and other contaminants. Alternatively, an AHF fraction may be obtained from plasma using solid phase polyelectrolyte resins. High purity concentrates of factor VIII may be produced from cryoprecipitate extracts by fractional precipitation with polyethylene glycol (PEG), PEG and glycine, and ethanol. In general, between about 200 and 400 units (by activity) of intermediate purity AHF concentrate may be recovered from a liter of blood, while between 100 and 250 units per liter of high purity concentrates can be obtained. The pro-coagulant activity of these concentrates (which is substantially lost when contrasted with the higher levels of AHF activity initially present in the plasma from which they were obtained) may be substantially restored and enhanced by incubation with manganese salts according to the present invention. Using the present invention, effective AHF activity levels have been restored to cryoprecipitate samples in which the AHF activity level has been completely lost (i.e. to 0 units of activity by exposure to metal chelating agents). Addition of manganese salts to plasma, plasma fractions or cryoprecipitate, and incubation under the temperature and pH conditions described above can also be used to increase the factor VIII activity yield from current levels (between about 40-60 percent activity) by a factor of approximately 50 percent to levels between about 70-90 percent activity.

The manganese restoration principle of the present invention has been confirmed by ESR (electron spin resonance spectroscopy) and may be widely applied to other aspects of factor VIII blood chemistry. Thus, manganese + + salts can be used as an adjuvant to the anti-coagulant compositions presently employed in the collection of whole blood to minimize the loss of labile factor VIII activity. In this aspect of the invention Mn + + salts in the range of 0.001 - 0.5 molar may be added to the anti-coagulant (e.g., sodium citrate) solution present in the whole blood collection container.

Factor VIII has also been implicated as being useful in the treatment of VonWillebrands disease. While the exact composition of the protein component of the factor VIII molecule is not available, the EDTA treated (pro-coagulant inactive) cryoprecipitate has been separated into two fractions (low molecular weight and high molecular weight proteins). The high molecular weight fraction has been identified as VonWillebrands factor. By coupling the process of the present invention with metal chelating treatments (such as EDTA) of factor VIII, it is possible to separate the relatively low molecular weight coagulant inactive protein from the high molecular weight VonWillebrand factor by affinity chromotography, gel filtration, dialysis, high pressure liquid chromotography (HPLC) or Amicon type filtration. The low molecular weight factor VIII protein may be incubated with manganese salts to synthesize a pro-coagulant active factor VIII metallo protein. This provides more potent factor VIII per unit of protein employed for therapeutic purposes and reduces the risk of hepatitus and immunologically negative reactions.

The present invention makes it possible to employ EDTA or other chelating agents to destroy contaminating viruses such as hepatitis often present in plasma fractions. The factor VIII pro-coagulant activity destroyed by exposure to EDTA may be regenerated by incubation with manganese salts to afford a factor VIII product that has a significantly lower risk of hepatitis attendant to its clinical use.

The technique for cryoprecipitation of whole blood for the production of AHF concentrates are discussed in detail in articles by: A.J. Johnson et al, Thromb. Diath. Haemorrh. Suppl. 35, 49 (1969); J. Newman et al., British Journal of Hematology, Volume 21, page 1 (1971), and H.L. James and M. Wickerhouser, Vox Sang, 23, 402 (1972). The technique for obtaining AHF (Factor VIII) fractions from plasma using solid phase polyelectrolyte resins are discussed in detail in an article by A.J. Johnson et al., J. Lab. Clin. Med., Volume 92, pp. 192-210 (1978).

The principle upon which the present invention operates is believed to be that EDTA and other metal chelating anti-coagulant agents decompose factor VIII by chelating manganese + + from the factor VIII molecule thereby destroying its pro-coagulant activity. In total plasma and its fractions, the removal of manganese ++ renders the materials clinically deficient with respect to factor VIII activity.

The invention will be further described with reference to the following examples:

## Example I

A purified human factor VIII (cryoprecipitate) having an activity of 1.5 units per mililiter was incubated in solutions containing respectively 45 millimolar EDTA, 45 millimolar EGTA and water, at room temperature overnight. The resulting solutions were dialyzed four hours in .34% citrate, pH7.4 and assayed for clotting activity.

|        | U/ml | % activity remaining |
|--------|------|----------------------|
| EDTA   | nil  | 0                    |
| EGTA   | 0.42 | 30                   |
| $H_2O$ | 1.38 | 91                   |

The results of this test show that there is a substantial loss of factor VIII activity attendant upon incubation with EDTA and less with EGTA.

## Example II

An attempt was undertaken to regenerate factor VIII activity by incubating the inactive EDTA treated material of Example I with manganese (++) chloride salts at 4 degrees centigrade for 48 hours. The result of this assay (summarized in the table below) revealed that manganese chloride is effective in regenerating lost factor VIII activity.

| Salt     | mM Conc. | F VIII Activity U/ml |
|----------|----------|----------------------|
| $MnCl_2$ | 17       | 1.10                 |
| $MnCl_2$ | 8.5      | 0.65                 |
| $H_2O$   | --       | nil                  |

## Example III

The EDTA human treated factor VIII described above (Example I) was tested in a conventional one stage PTT clotting assay, and in thrombin activation clotting assay. The assays verified the complete loss of factor VIII activity. Overnight incubation of the inactive material with calcium chloride (0.02 molar) at 4 degrees centigrade had no effect on clotting activity. A significant increase in clotting activity from 0 to 0.25 units per mililiter was obtained by overnight incubation with manganese chloride (0.02 molar) at 4 degrees centigrade. This regenerated factor VIII activity was increased to 17 units/mililiters by activation with 10 ng/m1 of thrombin.

Conditions: EDTA treated human factor VIII was incubated in 17 mM metal salts at 4 degrees centigrade overnight. Thrombin activation was carried out using 10ng/ml purified bovine thrombin, 37 degrees centigrade for 1 min., at which time an aliquot was removed for clotting assays.

| Salt | Clotting, U/m1 | Thrombin Activation |
|---|---|---|
| $MnCl_2$ | 0.25 | 17 |
| $CaCl_2$ | nil | nil |
| $H_2O$ | nil | nil |

The results of this test comprise evidence that EDTA acts to chelate manganese from active factor VIII to render the protein inactive. Subsequent synthesis to active factor VIII metallo-protein follows incubation with a manganese (++) salt, preferably manganese chloride $(MnCl_2)$.

### Example IV

The following test was conducted to obtain a partial time course of regeneration of lost factor VIII activity. EDTA human factor VIII (Example I) as dialyzed in citrate buffer and incubated with 17 millimolar (0.017 molar) manganese chloride at different temperatures. Aliquots were removed at timed intervals and assayed for clotting activity and thrombin activation. Parallel assays were run at room temperature and 37 degrees centigrade. The results of the assays are presented in the table VI below.

## TABLE VI

| Incubation Time, Min. | Room Temp. Incubation | | 37 Degrees Incubation | |
|---|---|---|---|---|
| | Units/ml | Units/ml, Thrombin Activation | Units/ml | Units/ml, Thrombin Activation |
| 0 | nil | nil | nil | nil |
| 10 | nil | nil | .06 | .43 |
| 30 | nil | nil | .12 | .73 |
| 40 | .04 | .20 | .14 | .73 |
| 60 | .06 | .21 | .17 | .94 |
| 80 | .07 | .36 | .17 | 1.04 |

The results of these tests indicate that synthesis of factor VIII pro-coagulant activity from pro-coagulant inactive protein occurs more rapidly at 37 degrees centigrade. Within an 80 minute incubation period, activity rose from 0 to 0.7 units per mililiter. This material was thrombin activatable to 1.04 units per mililiter. Synthesis was slower at room temperature. A control experiment using water instead of manganese chloride provided no generation of activity.

## Example V

Purified bovine factor VIII (obtained according to the procedure of Schmer et al in "The Isolation and Characterization of Bovine Factor VIII", Journal of Biological Chemistry Volume 247 pp. 2512-2421 (1972)) having an activity level of 3.3 units per mililiter and being eightfold thrombin activatable was incubated with 27 milimolar EDTA at 4 degrees centigrade overnight and dialyzed against 0.34% citrate buffer (pH7.4). Clotting assays revealed that the EDTA treated factor VIII had completely lost its activity and was not thrombin activatable. Clotting activity was generated from 0 to 1.4 units per mililiter by incubating with manganese chloride (0.02 molar) at 37 degrees centigrade for 80 minutes. Aliquots of the manganese chloride incubated material were withdrawn at timed intervals and assayed for clotting activity. The generated factor VIII was thrombin activatable to 27 units per mililiter.

| Incubation Time, Min. | Clot Units U/ml | Thombin Activation U/ml |
|---|---|---|
| 0 | nil | nil |
| 10 | .86 | 9.5 |
| 20 | .90 | 12.6 |
| 40 | .99 | 16.7 |
| 50 | 1.10 | 17.2 |
| 60 | 1.30 | 22.5 |
| 80 | 1.40 | 27.0 |

A control experiment using water instead of manganese chloride provided no generation of activity.

### Example VI

A commercially obtained sample of lyophilized factor VIII concentrate (New York Blood Center) (Activity = 1.30 U/ml) was dissolved in water. Aliquots of this material were incubated for one hour at 37°C with small amounts of water, and solutions of $MnCl_2$ and $CaCl_2$ (both giving final concentrations of 0.02 Molar). Factor VIII procoagulant clothing activities were measured as shown below.

| Sample + Adjuvant | mg/ml Protein Concentration | U/ml Clot Activity | U/ml Net Change In Activity |
|---|---|---|---|
| comm'l factor VIII + H$_2$O | 22.1 | 1.20 | -.10 |
| "       "       " + CaCl$_2$(20mM) | 22.1 | 1.40 | +.10 |
| "       "       " + MnCl$_2$(20mM) | 22.1 | 2.20 | +.90 |

Similar results were obtained from the cryoprecipitate derived from frozen and thawed normal human blood plasma collected in citrate dextrose (activity = 0.42 U/ml) and incubated for one hour at 37°C.

| Sample + Added | mg/ml Protein Concentration | U/ml Clot Activity | U/ml Net Change In Activity |
|---|---|---|---|
| human cryoprecepitate | | | |
| " + $H_2O$ | 18.1 | 0.40 | -.02 |
| " + $CaCl_2$(0.02M) | 18.1 | 0.45 | +.03 |
| " + $MnCl_2$(0.02M) | 18.1 | 0.90 | +.48 |

From the foregoing, it will be seen that the present invention provides a technique for synthesizing lost factor VIII activity from various blood fractions.

The EDTA sodium salt, EGTA and purified bovine thrombin used in these examples were purchased from Sigma Chemical Company, St. Louis, Mo. One stage prothrombin time clotting assays were conducted using bovine plasma as a standard having a 1 unit per mililiter factor VIII activity.

WHAT IS CLAIMED IS:

1. A method for generating factor VIII pro-coagulant activity in a mammal derived plasma fraction from which said activity has been at least partially lost which comprises incubating said fraction in the presence of an effective amount for regenerating factor VIII pro-coagulant activity of a pharmaceutically acceptable divalent manganese salt.

2. The method according to claim 1 wherein said divalent metal salt comprises a manganese halide.

3. The method of claim 2 wherein said halide salt is manganese chloride.

4. The method of claim 3 which comprises incubating said blood product in the presence of said salt for a predetermined time period.

5. The method of claim 4 which comprises conducting said incubating step at a temperature of between about 4 degrees centigrade and 60 degrees centigrade.

6. The method of claim 5 which comprises conducting said incubation at between about pH3 and pH8.5.

7. The method of claim 6 which comprises conducting said incubation in the presence of a buffer solution having a a concentration of up to about 0.5 molar.

8. The method of claim 7 wherein said manganese salt has a concentration range of between about 0.001 and 0.5 molar.

9. The method of claim 6 wherein said blood product comprises a cryoprecipitate.

10. The method of claim 9 which comprises obtaining said cryoprecipitate from whole blood collected in the presence of a metal chelating anti-coagulant agent.

11. The method of claim 10 wherein said anti-coagulant agent comprises sodium citrate.

12. A blood product comprising a cryoprecipitate derived from human plasma, said cryoprecipitate containing a pharmaceutically acceptable divalent manganese chloride salt in a concentration range between 0.001 and 0.5 molar.

13. The product of claim 12 wherein said salt comprises a manganese halide.

14. The product of claim 13 wherein said halide salt is manganese chloride.

15. A method for producing potent factor VIII pro-coagulant material substantially free of hepatitis contaminants which comprises:

preparing a blood plasma derived cryoprecipitate from whole blood collected in the presence of ethylene diamine tetra acetec acid,

separating said cryoprecipitate into high and low molecular weight fractions, said low molecular weight fraction containing a pro-coagulant inactive protein, and

incubating said low molecular weight fraction in the presence of an effective amount for regenerating pro-coagulant factor VIII activity of a divalent manganese salt.

16. A method of synthesizing a metallo-protein possessing Factor VIII pro-coagulant activity which comprises the steps of:

collecting mammalian blood in the presence of a metal chelating anti-coagulant,

separating plasma from the collected blood,

fractionating the plasma by using solid phase polyelectrolyte resins to obtain an AHF fraction, and

incubating the polyelectrolyte resin AHF fraction in the presence of an effective amount for generating metallo-protein having pro-coagulant Factor VIII activity of a divalent manganese salt.

17.   A method synthesizing a metallo-protein possessing Factor VIII pro-coagulant activity which comprises the steps of:

collecting mammalian blood in the presence of a metal chelating anti-coagulant,

separating plasma from the collected blood,

freezing the plasma,

thawing the plasma to form a cryoprecipitate fraction,

incubating the cryoprecipitate fraction in the presence of an effective amount for generating metallo-protein having pro-coagulant Factor VIII activity of a divalent manganese salt.

| | **DOCUMENTS CONSIDERED TO BE RELEVANT** | | **CLASSIFICATION OF THE APPLICATION (Int. Cl. 3)** |
|---|---|---|---|
| Category | Citation of document with indication, where appropriate, of relevant passages | Relevant to claim | |
| A | DE - A - 2 906 571 (L. LINCE MORA et al.) | | A 61 K 35/14 37/14 |
| | -- | | |
| A | GB - A - 2 012 162 (DUNCAN LEE McCOLLESTER) | | |
| | ---- | | |

**TECHNICAL FIELDS SEARCHED (Int.Cl. 3)**

A 61 K 35/00
33/00
37/00
A 01 N 1/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 15-02-1982 | MARIE |

EPO Form 1503.1 06.78